(19) Europäisches Patentamt / European Patent Office / Office européen des brevets

(11) EP 2 389 936 B1

(12) **EUROPEAN PATENT SPECIFICATION**

(45) Date of publication and mention of the grant of the patent:
**22.01.2014 Bulletin 2014/04**

(51) Int Cl.:
***A61K 31/245*** *(2006.01)* ***A61P 25/06*** *(2006.01)*

(21) Application number: **11166939.6**

(22) Date of filing: **20.05.2011**

(54) **Aminaphtone for use in the treatment and/or prevention of migraine**

Aminafton zur Vewerdung in der Behandlung und/oder Prävention von Migräne

Aminaphtone pour usage dans le traitement et/ou la prévention de la migraine

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **27.05.2010 IT MI20100958**

(43) Date of publication of application:
**30.11.2011 Bulletin 2011/48**

(73) Proprietor: **Laboratori Baldacci S.P.A.**
**56124 Pisa (IT)**

(72) Inventor: **Baldacci, MASSIMO**
**I-56124, Pisa (IT)**

(74) Representative: **Bartorelli, Luisa et al**
**Dragotti & Associati Srl**
**Via Nino Bixio, 7**
**20129 Milano (IT)**

(56) References cited:
**WO-A2-2007/116297**

- **HANSOTIA P: "Evaluation and treatment of headache. Practical approach to a common symptom.", POSTGRADUATE MEDICINE 1 MAY 1986 LNKD- PUBMED:3703765, vol. 79, no. 6, 1 May 1986 (1986-05-01), pages 75-79 , 82, XP009150591, ISSN: 0032-5481**
- **HAMED SHERIFA A: "The vascular risk associations with migraine: relation to migraine susceptibility and progression.", ATHEROSCLEROSIS JUL 2009 LNKD- PUBMED: 19054516, vol. 205, no. 1, July 2009 (2009-07), pages 15-22, XP002653109, ISSN: 1879-1484**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 389 936 B1

## Description

### State of the art

Migraine is a disabling disorder which involves a high percentage of individuals, for example, about 12% of the population in the USA suffers from migraine, and it usually appears in adults comprised between 20 and 40 years (see 8, incorporated herein by reference).

It is widely thought that migraine attacks are often caused by recurrent factors or situations and it is an indispensible basis for a good therapeutic approach to the disease, monitoring the patient, the symptoms thereof so as to supervise - for a few months - frequency, duration and intensity of the migraine attacks as well as the situations that triggered or favoured the beginning of the migraine pain.

Often, migraine attacks are accompanied by nausea, vomit, photophobia, phonophobia, hence the patient is forced to stay in bed and cannot perform any physical activity (see 9-10 incorporated herein by reference).

Currently, there is a high number of drugs that can intervene in various forms of migraine and the choice of the drug depends on the intensity of the pain, the duration and frequency of migraine attacks.

Among the most active drugs, 5-$HT_1$serotonergic receptor agonists are currently known, to be used in high intensity attacks; the ergot derivatives (dihydroergotamine and ergotamine) for the vasoconstricting activity thereof on the central vessels; substances provided with analgesic and anti-inflammatory activities whose action mechanism seems mainly related to the inhibition of the synthesis of the prostaglandins; β-blocker drugs and calcium antagonists in the treatment of particular forms of migraine.

These classes of drugs may have very serious adverse effects both at gastroenteric level such as for example nausea, vomit, abdominal pains, diarrhoea as well as at central nervous system level such as for example trembling, hypertonia, hypoesthesia/paresthesia, peripheral neuropathy, nervousness.

The treatment of this disorder, as indicated above, requires particular attention by the doctor when analysing both the type of life of the patient and the presence of other simultaneous diseases.

With reference to the diseases related to the therapeutic approach of migraine and the adverse effects - even serious - related to the use of drugs still available, there currently arises the need for new therapies for the treatment of migraine.

It has now been surprisingly discovered that aminaphtone, i.e. 2-hydroxy-3-methyl-1,4-naphthohydroquinone-2-p-aminobenzoate, and/or the pharmaceutically acceptable salts thereof, can be used in the treatment and/or prevention of migraine.

The pharmacological activity of aminaphtone, whose chemical formula is indicated below,

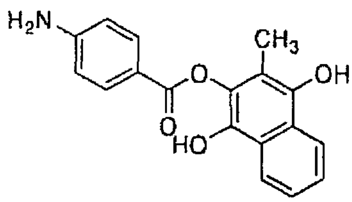

has been known over the years and the scientific literature reports studies which reveal the action of this compound on the venous capillary circulation as a modulator and/or normaliser of the alteration of the capillary vessels which occur in particular pathologic conditions (see 1,2,3,4 incorporated herein by reference).

Over the last years further studies have revealed the capacity of aminaphtone to antagonise pathological events related to an inflammatory state with alteration of the arterial vessel structure through biochemical mechanisms related to the production of endothelin and the hyper-expression of bonding molecules. Studies on human endothelial cells cultured in vitro revealed that the activity of aminaphtone is also present at very low concentrations (2-6 μg/ml) (see 5-6, incorporated herein by reference).

WO2007/116297 discloses the use of aminaphtone for heating arteriopathies of inflammatory type, in particular by reducing endothelial inflammation of arterial vascular system.

Hansotia P. Postgraduate Medicine 1986, 79(6), 75 describes the mechanisms of headache, in particular of vascular headache or migraine and the currently available pharmacotherapy for the treatment thereof.

Hamed Sherifa A. Atherosclerosis 2009, 205(1), 15 describes that migraine is associated with neurovascular inflammation. Said document also evaluates the comorbid vascular risk associations between migraine and other pathologies.

Other clinical evidence confirms that aminaphtone is efficient in the treatment the human endothelial inflammation, an inflammation at the base of degenerative diseases of the evolution type with loss of vessel integrity and formation of a potent endogenous vasoconstrictor such as endothelin (see 7, incorporated herein by reference).

The term "inflammatory vascular problems", according to the present invention, is used to indicate the diseases mentioned above.

Therapeutic use was verified and used in medical practice for the treatment of venous diseases.

The drug is well tolerated and no noticeable collateral effect was observed.

However, up to date there has never been observed any pharmacological property of aminaphtone in the treatment and/or prevention of migraine.

## Description of the invention

An object of the present invention is aminaphtone and/or pharmaceutically acceptable salts thereof for use in the treatment and/or prevention of migraine, preferably said migraine being generated by the inflammatory vascular problems, both of the venous and arterial type.

The results obtained and indicated hereinafter in the experimental part, allow including aminaphtone as a drug to be used in the treatment of the various forms of migraine, a disorder whose therapy is difficult to approach for the doctor due to the variety of physiopathogenic mechanisms it is based on.

Our experimental data using aminaphtone reveals that this drug is well tolerated even after long treatments.

According to the present invention, aminaphtone can be administered to human beings, both intended as an adult subject and as "paediatric population", where the term "paediatric population" is used to indicate the part of population between birth and eighteen years of age

According to the present invention, aminaphtone can be administered enterally, for example orally, sublingually, rectally or parenterally, for example through intravenous route, intramuscular route, inhalation route, topical route, subcutaneous route, preferably oral route.

According to the present invention, aminaphtone can be administered at an amount comprised between 50 and 150 mg per dose, preferably about 75 mg per dose. According to a preferred embodiment of the present invention, aminaphtone is administered two to four times per day, preferably three times per day, more preferably for at least 1 month, even more preferably for at least 2 months.

## Experimental part

The study was conducted on 6 patients aged between 40 and 60 years with at least a 2-year record of migraine attacks of various seriousness.

The following 4-point scale was used as efficiency parameters for the evaluation of aminaphtone on the treatment of migraine:

(0= no pain; 1= slight pain; 2= mild pain; 3= intense pain).

The drug is considered efficient if it shows a reduction of intensity of pain by at least one point of the scale.

The 6 patients were treated using 75 mg of aminaphtone three times per day and the treatment lasted for at least 1 month.

After 2 hours from the beginning of the oral administration, the percentage of patients revealing therapeutic benefit was evaluated and this evaluation was repeated 24 hours and 48 hours from the first administration, as represented in Figure 1.

After 2 hours, 4 treated patients out of 6 revealed a reduction of the intensity of pain by at least one point of the scale and after 48 hours from the beginning of the treatment all patients no longer had migraine attacks, including those considered slight pain.

Given the tolerability of the drug, therapy using aminaphtone continues up to date, after about 2 months from the beginning of the therapy, there were no migraine attacks. Bibliography


1) E. Gelso, R. Corradetti, "Etiopathogenesis of the chronic venous insufficiency: actuality of aminaphtone (Capillarema) in microcircle alterations", Intern. journal on drugs and therapy vol. XXI, 1-8, 2004.

2) N. Di Paolo, C. Bartoli, "Angioprotective action of aminaphtone on the immunological purpura with antiplatelet serum in the rabbit ear", Quad. Coag. 1974, 15, 67-71.

3) Villaverde C.A. et al., "Modification of the vascular permeability with Aminaphtone ", Rev. Farmacol. Clin. Exp. 1989, 6, 9-14.

4) Castelli P. et al., "Treatment with Aminaphtone in the complicated chronic venous insufficiency", Flebolinfologia 1988, 1, 241-44.
5) S. Lemma et al., "Novel mode of action of the Aminaphtone : down-regulation of a E-selective expression in ECV-304 cells", Int. Angiology 2006, 25, suppl 1, 189.
6) R. Scorza et al., "Aminaphtone, a derivative of 4-aminobenzoic acid, downregulates endothelin 1 production in ECV 304 cells: an in vitro study", Drugs in R.D. 2008, 9, 251-257.
7) R. Scorza et al., "Aminaphtone enhances iloprost beneficial effect in patients with systemic sclerosis and recurrent ulcers", 2009 ACR/ARHP Annal Scientific Meeting Philadelphia PA, October 16-21.
8) Loretta L. Mueller, "Diagnosis and Managing migraine Headache", J. Am. Osteopath As 2007, 107 (suppl. 6) 10-16.
9) G. Sandrini et al., "Neurophysiological tests and neuroimaging procedures in non acute headache: guidelines and recommendations", Europ. Journal of Neurology 2004, 11, 217-224.
10) S. Evers et al., "EFNS guideline on the drug treatment of migraine revised report of an EFNS task force", Europ. Journal of Neurology 2009, 16, 968-81.

## Claims

1. Aminaphtone and/or pharmaceutically acceptable salts thereof for use in the treatment and/or prevention of migraine.

2. Aminaphtone according to claim 1 for use in the treatment and/or prevention of migraine, **characterised in that** said migraine is generated by inflammatory vascular problems.

3. Aminaphtone according to claim 1 for use in the treatment and/or prevention of migraine, **characterised in that** it is administered enterally or parentally.

4. Aminaphtone according to claim 3 for use in the treatment and/or prevention of migraine, **characterised in that** it is administered orally.

5. Aminaphtone according to claim 1 for use in the treatment and/or prevention of migraine, **characterised in that** it is administered at an amount comprised between 50 and 150 mg per dose.

6. Aminaphtone according to claim 5 for use in the treatment and/or prevention of migraine, **characterised in that** it is administered at an amount of about 75 mg per dose.

7. Aminaphtone according to claim 1 for use in the treatment and/or prevention of migraine, **characterised in that** it is administered two to four times per day.

8. Aminaphtone according to claim 7 for use in the treatment and/or prevention of migraine, **characterised in that** it is administered three times per day.

9. Aminaphtone according to claim 7 for use in the treatment and/or prevention of migraine, **characterised in that** it is administered for at least one month.

10. Aminaphtone according to claim 9 for use in the treatment and/or prevention of migraine, **characterised in that** it is administered for at least two months.

## Patentansprüche

1. Aminafton und/oder pharmazeutisch akzeptable Salze davon zur Verwendung bei der Behandlung und/oder Prävention von Migräne.

2. Aminafton nach Anspruch 1 zur Verwendung bei der Behandlung und/oder Prävention von Migräne, **dadurch gekennzeichnet, dass** die Migräne durch vaskulare Enzündungsprobleme verursacht ist.

3. Aminafton nach Anspruch 1 zur Verwendung bei der Behandlung und/oder Prävention von Migräne, **dadurch gekennzeichnet, dass** es enteral oder parental verabreicht wird.

4. Aminafton nach Anspruch 3 zur Verwendung bei der Behandlung und/oder Prävention von Migräne, **dadurch gekennzeichnet, dass** es oral verabreicht wird.

5. Aminafton nach Anspruch 1 zur Verwendung bei der Behandlung und/oder Prävention von Migräne, **dadurch gekennzeichnet, dass** es in einer Menge verabreicht wird, die zwischen 50 und 150 mg pro Dosis enthalten ist.

6. Aminafton nach Anspruch 5 zur Verwendung bei der Behandlung und/oder Prävention von Migräne, **dadurch gekennzeichnet, dass** es in einer Menge von ungefähr 75 mg pro Dosis verabreicht wird.

7. Aminafton nach Anspruch 1 zur Verwendung bei der Behandlung und/oder Prävention von Migräne, **dadurch gekennzeichnet, dass** es zwei bis vier Mal pro Tag verabreicht wird.

8. Aminafton nach Anspruch 7 zur Verwendung bei der Behandlung und/oder Prävention von Migräne, **dadurch gekennzeichnet, dass** es drei Mal pro Tag verabreicht Wird.

9. Aminafton nach Anspruch 7 zur Verwendung bei der Behandlung und/oder Prävention von Migräne, **dadurch gekennzeichnet, dass** es wenigstens einen Monat lang verabreicht wird.

10. Aminafton nach Anspruch 9 zur Verwendung bei der Behandlung und/oder Prävention von Migräne, **dadurch gekennzeichnet, dass** es wenigstens zwei Monate lang verabreicht wird.

## Revendications

1. Aminaphtone et/ou sels pharmaceutiquement acceptables de celle-ci pour utilisation dans le traitement et/ou la prévention de la migraine.

2. Aminaphtone selon la revendication 1 pour utilisation dans le traitement et/ou la prévention de la migraine, **caractérisée en ce que** ladite migraine est due à des problèmes vasculaires inflammatoires.

3. Aminaphtone selon la revendication 1 pour utilisation dans le traitement et/ou la prévention de la migraine, **caractérisée en ce qu'**elle est administrée par voie entérale ou parentérale.

4. Aminaphtone selon la revendication 3 pour utilisation dans le traitement et/ou la prévention de la migraine, **caractérisée en ce qu'**elle est administrée par voie orale.

5. Aminaphtone selon la revendication 1 pour utilisation dans le traitement et/ou la prévention de la migraine, **caractérisée en ce qu'**elle est administrée en une quantité comprise entre 50 et 150 mg par dose.

6. Aminaphtone selon la revendication 5 pour utilisation dans le traitement et/ou la prévention de la migraine, **caractérisée en ce qu'**elle est administrée en une quantité d'environ 75 mg par dose.

7. Aminaphtone selon la revendication 1 pour utilisation dans le traitement et/ou la prévention de la migraine, **caractérisée en ce qu'**elle est administrée deux à quatre fois par jour.

8. Aminaphtone selon la revendication 7 pour utilisation dans le traitement et/ou la prévention de la migraine, **caractérisée en ce qu'**elle est administrée trois fois par jour.

9. Aminaphtone selon la revendication 7 pour utilisation dans le traitement et/ou la prévention de la migraine, **caractérisée en ce qu'**elle est administrée pendant au moins un mois.

10. Aminaphtone selon la revendication 9 pour utilisation dans le traitement et/ou la prévention de la migraine, **caractérisée en ce qu'**elle est administrée pendant au moins deux mois.

Figure 1

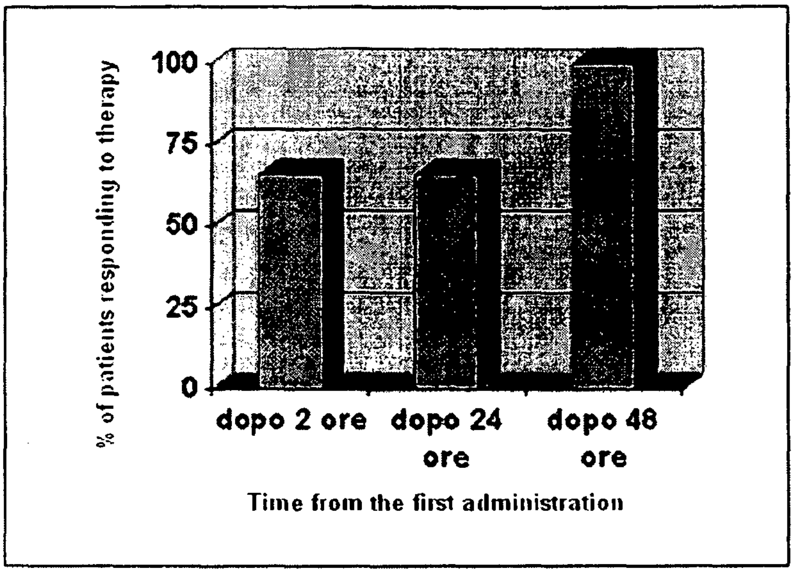
% of patients responding to therapy
100
75
50
25
0
dopo 2 ore
dopo 24 ore
dopo 48 ore
Time from the first administration

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007116297 A


### Non-patent literature cited in the description


- **HANSOTIA P.** *Postgraduate Medicine,* 1986, vol. 79 (6), 75
- **HAMED SHERIFA A.** *Atherosclerosis,* 2009, vol. 205 (1), 15
- **E. GELSO ; R. CORRADETTI.** Etiopathogenesis of the chronic venous insufficiency: actuality of aminaphtone (Capillarema) in microcircle alterations. *Intern. journal on drugs and therapy,* 2004, vol. XXI, 1-8
- **N. DI PAOLO ; C. BARTOLI.** Angioprotective action of aminaphtone on the immunological purpura with antiplatelet serum in the rabbit ear. *Quad. Coag.,* 1974, vol. 15, 67-71
- **VILLAVERDE C.A. et al.** Modification of the vascular permeability with Aminaphtone. *Rev. Farmacol. Clin. Exp.,* 1989, vol. 6, 9-14
- **CASTELLI P. et al.** Treatment with Aminaphtone in the complicated chronic venous insufficiency. *Flebolinfologia,* 1988, vol. 1, 241-44
- **S. LEMMA et al.** Novel mode of action of the Aminaphtone : down-regulation of a E-selective expression in ECV-304 cells. *Int. Angiology,* 2006, vol. 25 (1), 189
- **R. SCORZA et al.** Aminaphtone, a derivative of 4-aminobenzoic acid, downregulates endothelin 1 production in ECV 304 cells: an in vitro study. *Drugs in R.D,* 2008, vol. 9, 251-257
- **R. SCORZA et al.** Aminaphtone enhances iloprost beneficial effect in patients with systemic sclerosis and recurrent ulcers. *ACR/ARHP Annal Scientific Meeting Philadelphia PA,* 16 October 2009
- **LORETTA L. MUELLER.** Diagnosis and Managing migraine Headache. *J. Am. Osteopath As,* 2007, vol. 107 (6), 10-16
- **G. SANDRINI et al.** Neurophysiological tests and neuroimaging procedures in non acute headache: guidelines and recommendations. *Europ. Journal of Neurology,* 2004, vol. 11, 217-224
- **S. EVERS et al.** EFNS guideline on the drug treatment of migraine revised report of an EFNS task force. *Europ. Journal of Neurology,* 2009, vol. 16, 968-81